# EUROPEAN PATENT SPECIFICATION

(11) **EP 2 231 559 B1**
(45) Date of publication and mention of the grant of the patent: **23.05.2012**
(21) Application number: 08859714.1
(22) Date of filing: 27.11.2008
(51) Int. Cl.: C07C 1/24, C07C 15/46

(54) **PROCESS FOR THE PREPARATION OF STYRENE AND/OR A SUBSTITUTED STYRENE**
VERFAHREN ZUR HERSTELLUNG VON STYREN UND/ODER EINEM SUBSTITUIERTEN STYREN
PROCEDE DE PREPARATION DU STYRENE ET/OU D'UN STYRENE SUBSTITUE

(30) Priority: 10.12.2007 EP 07122779
(43) Date of publication of application: 29.09.2010
(73) Proprietor: Shell Internationale Research Maatschappij B.V., 2596 HR The Hague (NL)
(72) Inventor: BOS, Alouisius, Nicolaas, Renee, NL-1031 CM Amsterdam (NL); KORADIA, Pramod, B., Hudson Ohio 4436 (US)
(74) Representative: Matthezing, Robert Maarten
(86) International application number: PCT/EP2008/066353
(87) International publication number: WO 2009/074461

(56) References cited:
- WO-A-99/58480
- R.G. ROMANOV ET AL: "Catalytic Dehydration of alpha-Phenylethanol on the Surface of Aluminium Oxides" KINETICS AND CATALYSIS, vol. 45, no. 3, 2004, pages 423-428, XP009099808
- G. VIVEKANANDAN AND V. KRISHNASAMY: "CATALYTIC TRANSFORMATIONS OF PHENETHYL ALCOHOLS IN THE VAPOUR PHASE" HUNGARIAN JOURNAL OF INDUSTRIAL CHEMISTRY, vol. 23, 1995, pages 21-26, XP009099987

## Description

The present invention relates to a process for the preparation of styrene and/or a substituted styrene from a feed containing 1-phenylethanol and 2-phenylethanol and/or a substituted 1-phenylethanol and a substituted 2-phenylethanol. 1-Phenylethanol is also known as alpha-phenylethanol or methylphenylcarbinol. 2-Phenylethanol is also known as beta-phenylethanol.

A commonly known method for manufacturing styrene is the coproduction of propylene oxide and styrene starting from ethylbenzene. In general such process involves the steps of (i) reacting ethylbenzene with oxygen or air to form ethylbenzene hydroperoxide, (ii) reacting the ethylbenzene hydroperoxide thus obtained with propene in the presence of an epoxidation catalyst to yield propylene oxide and 1-phenylethanol, and (iii) converting the 1-phenylethanol into styrene by dehydration using a suitable dehydration catalyst. In step (ii) of said process, 2-phenylethanol is formed as a by-product which is also converted into styrene in step (iii).

The production of styrene by dehydrating 1-phenylethanol (and 2-phenylethanol) is well known in the art. It can be carried out both in the gas phase and in the liquid phase. The present invention is directed to gas phase dehydration. The use of alumina catalysts in such a gas phase dehydration is well known in the art.

WO-A-99/58480 (in the name of the present applicant) describes a process for the preparation of styrene comprising the gas phase dehydration of 1-phenylethanol at elevated temperature in the presence of a dehydration catalyst consisting of shaped alumina catalyst particles having a surface area in the range of from 80 to 140 m²/g and a pore volume in the range of from 0.35 to 0.65 ml/g, of which 0.03 to 0.15 ml/g is in pores having a diameter of at least 1,000 nm.

WO-A-00/25918 (in the name of Engelhard Corp.) describes star shaped alumina extrudates with a pore volume in pores of diameter of over 1,000 nm of at least 0.05 ml/g, a side crushing strength of at least 50 N and a bulk crushing strength of at least 1 MPa.

WO-A-2004/076389 (in the name of the present applicant) describes a process for the preparation of styrene comprising the gas phase dehydration of 1-phenylethanol at elevated temperature in the presence of a dehydration catalyst comprising shaped alumina catalyst particles having a surface area of from 80 to 140 m²/g. Said process is characterised in that the catalyst pore volume is more than 0.65 ml/g.

It is desired to use a catalyst in the gas phase dehydration, which catalyst would maintain a high activity and selectivity after the reaction has started, that is to say after aging of the catalyst.

Dehydration of 2-phenylethanol to styrene is slower than dehydration of 1-phenylethanol. In addition, the conversion rate for 2-phenylethanol tends to decrease quicker in time than the conversion rate for 1-phenylethanol. 2-Phenylethanol may be a source for heavy ends formation. Heavy ends comprise heavy by-products like oligomers and ethers.

Therefore, an object of this invention is to provide a process for the preparation of styrene allowing high activity and selectivity in the conversion of 1-phenylethanol and 2-phenylethanol to styrene, also after the catalyst has aged. In addition, the catalyst to be used should have sufficient mechanical strength.

It has been found that 1-phenylethanol and 2-phenylethanol can be converted with a sufficient activity into styrene for a prolonged period of time, when a catalyst having a multimodal pore size distribution is used.

Accordingly, the present invention relates to a process for the preparation of styrene and/or a substituted styrene from a feed containing 1-phenylethanol and 2-phenylethanol and/or a substituted 1-phenylethanol and a substituted 2-phenylethanol, comprising a gas phase dehydration of the feed at elevated temperature in the presence of a catalyst comprising particles of alumina having a multimodal pore size distribution.

It has been found that with the present process 2-phenylethanol can advantageously be converted with a sufficient activity into styrene for a prolonged period of time, whilst a high activity for the conversion of 1-phenylethanol into styrene is maintained.

In accordance with the present invention, the feed for the gas phase dehydration may be a feed containing a substituted 1-phenylethanol and a substituted 2-phenylethanol, thereby producing a substituted styrene. With "substituted styrene" is meant a styrene containing one or more substituents bonded to the aromatic ring and/or to the vinyl group. Such substituents typically include alkyl groups, such as C₁-C₄ alkyl groups, for example methyl and ethyl groups. The substituents of the substituted styrene, the substituted 1-phenylethanol and the substituted 2-phenylethanol are all identical. An example of a substituted styrene which can be prepared according to the present process, is alpha-methyl-styrene to be prepared from a feed containing 1-methyl-1-phenylethanol and 2-methyl-2-phenylethanol.

The term "alumina" as used in connection with the present invention refers to an inorganic oxide consisting for at least 90% by weight (wt%), preferably at least 95 wt% and most preferably at least 99 wt%, of Al₂O₃. The remainder up to 100 wt% may consist of minor amounts of other inorganic oxides like SiO₂ and alkali metal oxides. Preferably no such other inorganic oxides are present and an inorganic oxide consisting of essentially 100 wt% of alumina is used. Suitable aluminas include gamma-alumina, delta-alumina, eta-alumina, theta-alumina, chi-alumina and kappa-alumina. Suitable alumina raw materials include alumina monohydrate (boehmite), alumina trihydrate (gibbsite, bayerite), transition alumina, or mixtures of the above.

The alumina catalyst to be used in the process of the present invention preferably has a surface area in the range of from 60 to 160 m²/g, more preferably in the range of from 80 to 140 m²/g. Still more preferably, the surface area of the catalyst is in the range of from 85 to 115 m²/g. The surface area is determined according to the well known Brunauer-Emmett-Teller (BET) method.

Preferably the total pore volume of the catalyst is of from 0.25 to 1.50 ml/g, more preferably 0.5 to 1.25 ml/g. Still more preferably, the total pore volume is greater than 0.7 ml/g. The total pore volume is determined according to the well known mercury porosimetry method.

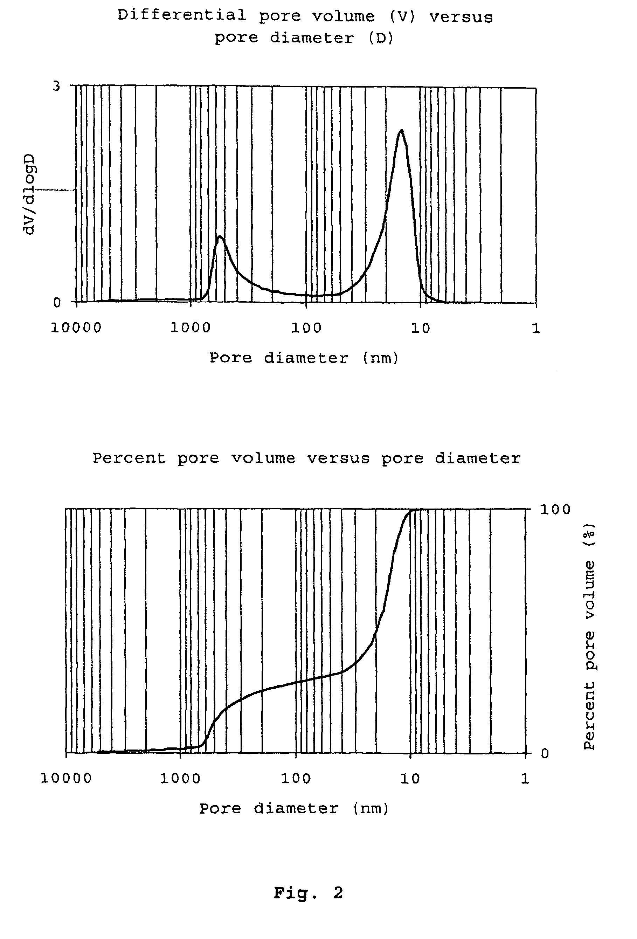

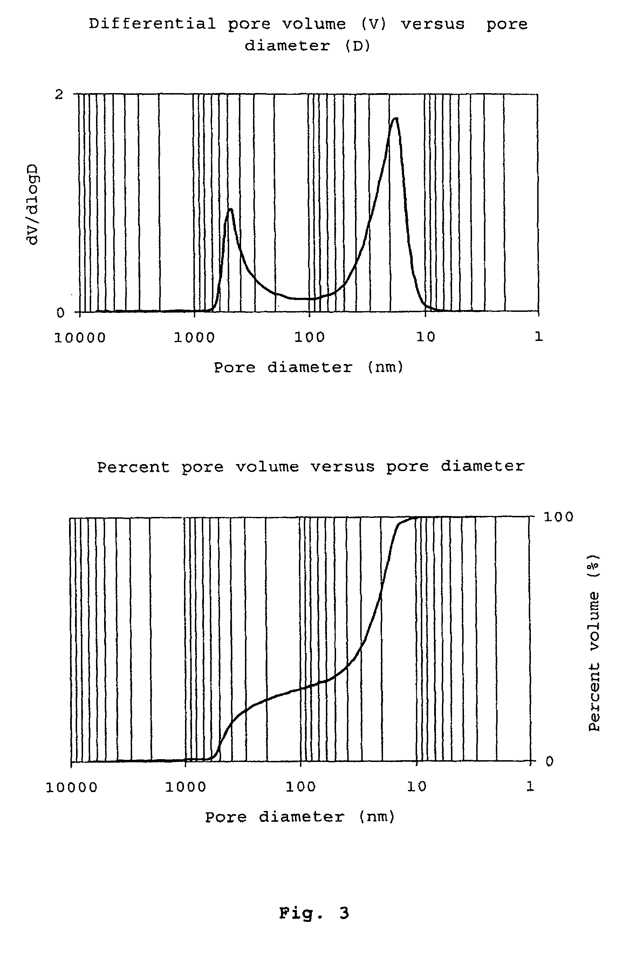

The catalyst to be used in the present invention has a multimodal pore size distribution. In accordance with this specification, a multimodal pore size distribution means a pore size distribution in which, when incremental pore volume is plotted as a function of pore size, the resulting function exhibits a maximum (or mode) within a first pore size range and a maximum (or mode) within a second pore size range. In general, a maximum (or mode) is the most frequently occurring number within a specific range of numbers. In relation to pore size distribution, the pore size maximum (or mode) is the pore size which, within a specific pore size range or within a subrange falling within such range, corresponds to the highest peak in a graph showing the pore size distribution. Therefore, in accordance with this specification, a multimodal pore size distribution means that within said first pore size range there should be at least one peak in a graph showing the pore size distribution, and within said second pore size range there should also be at least one peak in a graph showing the pore size distribution. Examples of multimodal pore size distributions having two peaks are shown in Figures 2 and 3. The pore size may be the pore diameter or the pore radius.

Preferably, in the multimodal pore size distribution, the pore size range comprises a first pore size range and a second pore size range and the pore sizes in the first pore size range are smaller than the pore sizes in the second pore size range.

Preferably a first pore size range is a pore diameter range of from 2 to 100 nm (mesopores) and a second pore size range is a pore diameter range of greater than 100 nm, for example greater than 100 nm to smaller than 10,000 or 1,000 nm (macropores). Preferably the maximum (or mode) in the first pore size range is at a pore diameter of from 5 to 30 nm, more preferably 10 to 20 nm. Further, preferably the maximum (or mode) in the second pore size range is at a pore diameter of from 300 to 1,000 nm, more preferably 400 to 700 nm.

Preferably the pore diameters corresponding to the maximums (or modes) in first and second pore size ranges are separated by at least 200 nm, more preferably at least 300 nm, and by at most 1,000 nm, more preferably at most 750 nm.

The median pore diameter calculated by volume (MPD_{V}) may be from 5 to 50 nm, preferably 10 to 40 nm and more preferably 15 to 30 nm. MPDᵥ herein means the pore diameter above which half of the total pore volume exists. Preferably the MPDᵥ is greater than the pore diameter mode in a first pore size range and smaller than the pore diameter mode in a second pore size range.

The pore size distribution is determined according to the well known mercury porosimetry method.

Preferably the catalyst to be used in the present invention has from 10 to 40%, more preferably 20 to 35%, and most preferably 25 to 30%, of the total pore volume in pores having a diameter greater than 100 nm (macropores). Further, preferably the catalyst has from 60 to 90%, more preferably 65 to 80%, and most preferably 70 to 75%, of the total pore volume in pores having a diameter from 2 to 100 nm (mesopores). Still further, preferably the catalyst has less than 3%, more preferably less than 2% and even more preferably less than 1%, of the total pore volume in pores having a diameter greater than 1,000 nm. Most preferably, the catalyst has essentially no pore volume in pores having a diameter greater than 1,000 nm.

The diameter of the catalyst particles is not particularly critical to the present invention. Diameters normally used for this kind of catalysts may be employed. The term "diameter" as used in this connection refers to the largest distance between two opposite points on the perimeter of the cross-section of a catalyst particle. In case of rod-like particles having a shaped cross-section, this shaped cross-section is the relevant cross-section. It has been found particularly advantageous for the purpose of the present invention to use catalyst particles having a diameter of 1.5 to 10 mm, preferably 2.5 to 7.5 mm.

In a preferred embodiment a shaped catalyst is used. The expression "shaped catalyst" refers to a catalyst having a certain spatial shape. Suitably shaped catalyst particles can be obtained by a method involving extrusion and calcination, wherein the spatial shape of the particles is obtained by using an extruder having a dieplate with an orifice of the desired shape. Generally, such shaping process comprises mixing one or more alumina raw materials with water or an acid solution to form an extrudable paste, forcing the paste through said orifices, cutting the extrudate to the desired length, and drying and calcining the formed pieces.

The catalyst particles may have any shape, including spherical, cylindrical, trilobal (three lobes), quadrilobal (four lobes), star-shaped, ring-shaped, cross-shaped etc. A star-shaped catalyst may comprise rod-like catalyst particles having a star-shaped cross-section. The star may have any desirable number of corners, but a four-, five- or six-cornered star-shape is preferred. Star-shaped objects can be defined as objects having some kind of central part or core, with three or more triangularly shaped extensions on the circumference thereof. An example of a star-shaped object is shown in the Figure of WO-A-00/25918.

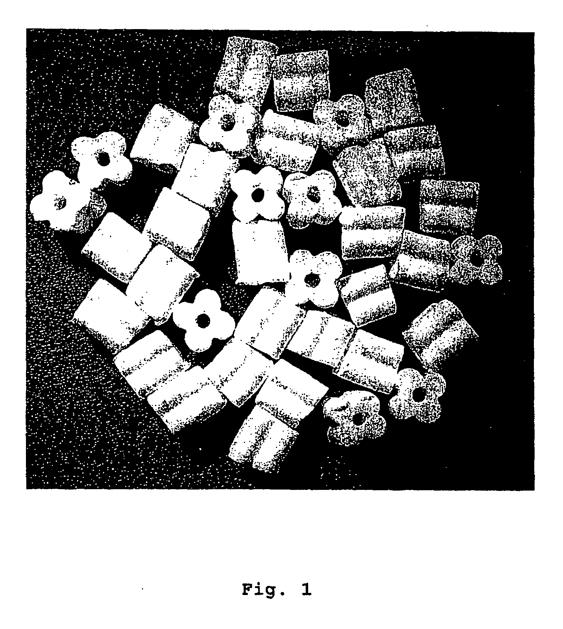

It has been found particularly advantageous to use a hollow quadrilobal shaped catalyst. A hollow quadrilobal shaped catalyst may comprise rod-like catalyst particles having a hollow quadrilobal shaped cross-section. By a hollow quadrilobal shaped cross-section is understood a cross-section having a central part which is at least partially hollow, with four non-triangularly, for example semi-circularly, shaped extensions on the circumference thereof. An example of a hollow quadrilobal shaped object is shown in Figure 1.

It has been found particularly advantageous to use a shaped catalyst having an average length/diameter ratio of the catalyst particles in the range of from 0.5 to 3, preferably 1.0 to 2.0. The "length" in this connection refers to the length of the rod of rod-like catalyst particles.

The catalyst particles to be used should also have sufficient mechanical strength. One of the advantages of a hollow "quadrilobal" shaped catalyst is that the catalyst particles still have a good mechanical strength despite the inner hole, both in terms of side crushing strength (SCS) and bulk crushing strength (BCS). Accordingly, the catalyst particles may have a SCS of at least 30 N, preferably at least 50 N, and a BCS of at least 0.7 MPa, preferably at least 1.0 MPa. For definitions of and methods of determining SCS and BCS reference is made to WO-A-00/25918.

Alumina catalysts and/or carriers having a multimodal pore size distribution and a desired specific surface area can be prepared by starting with a high surface area multimodal alumina carrier, and calcining to a suitable temperature to produce the desired specific surface area. For example, high surface area alumina carrier commercially available from Saint-Gobain NorPro of Stow, Ohio, USA and identified as SA6x76, can be fired to a temperature in the range of from 900 to 1060 °C to produce a specific surface area in the range of from 80 to 140 m²/g.

Alumina catalysts and/or carriers having a monomodal pore size distribution and a desired specific surface area can be prepared by starting with a high surface area monomodal alumina carrier, and calcining to a suitable temperature to produce the desired specific surface area. For example, high surface area alumina carrier commercially available from Saint-Gobain NorPro of Stow, Ohio, USA and identified as SA6x75, can be fired to a temperature in the range of from 900 to 1060 °C to produce a specific surface area in the range of from 80 to 140 m²/g.

The dehydration of 1-phenylethanol and 2-phenylethanol into styrene according to the present invention is carried out in the gas phase at elevated temperature. The term "elevated temperature" preferably is any temperature above 150 °C. The preferred dehydration conditions are those normally applied and include a reaction temperature in the range of from 210 to 330 °C, more preferably 280 to 320 °C, and a pressure in the range of from 0.1 to 10 bar, more preferably of about 1 bar.

The invention will now be illustrated by the following examples. In these examples the surface area is determined according to the BET method and the pore volume and the pore size distribution were determined according to the mercury porosimetry method. Further, in these examples the conversion of for example 1-phenylethanol is defined as the mole percentage of 1-phenylethanol converted relative to the total number of moles of 1-phenylethanol present in the feed. Still further, in this connection selectivity is defined as the mole percentage of styrene formed relative to the total number of moles of 1-phenylethanol and 2-phenylethanol converted.

### Examples and Comparative Examples

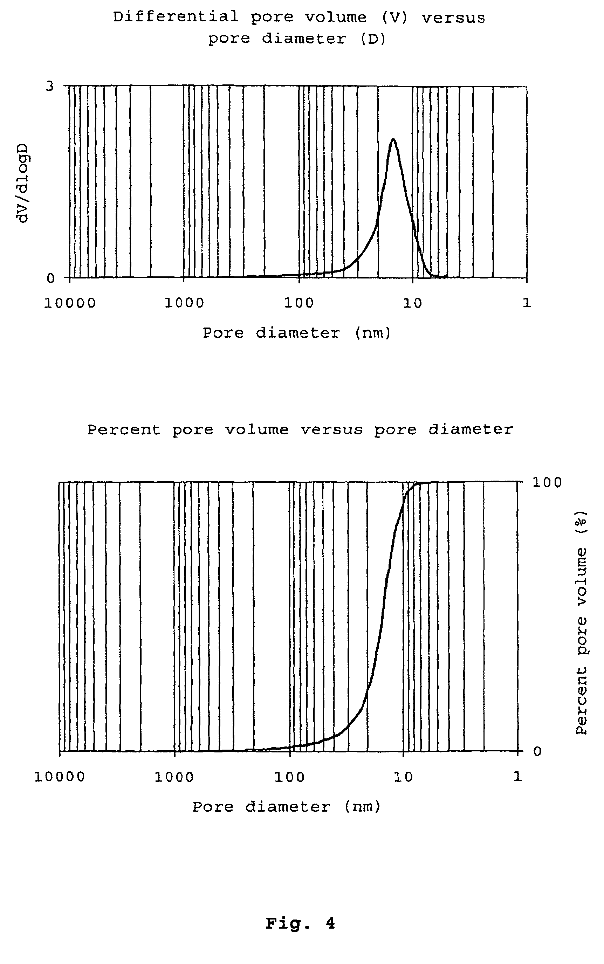

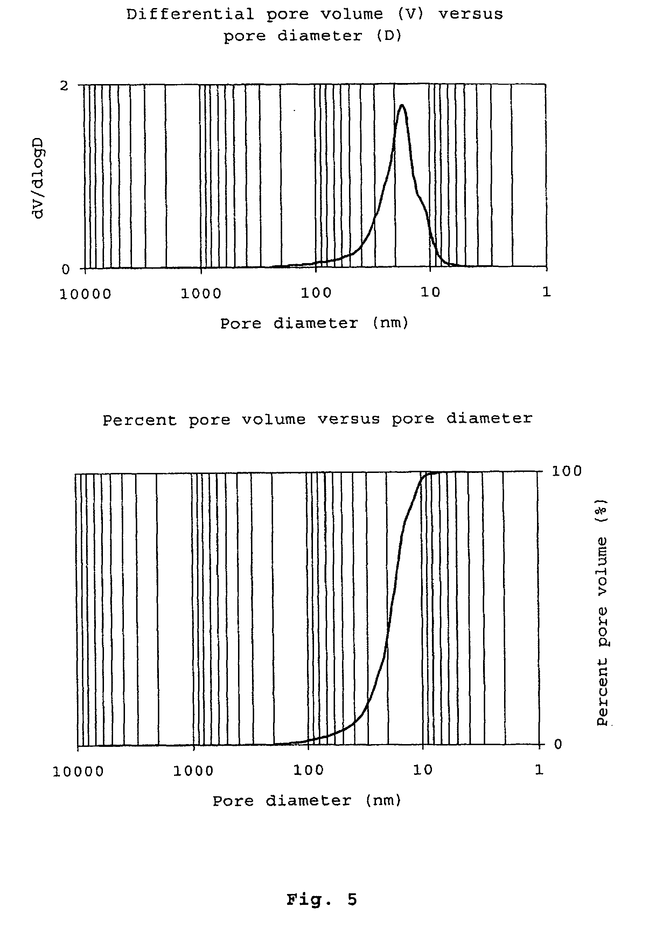

An alumina catalyst having the designation (A, B, C(*) or D(*)) and physical properties indicated in Table 1 and the pore size distribution shown in Figure 2, 3, 4 or 5, was tested for dehydration performance in a microflow unit consisting of a 13 mm diameter plugflow reactor, 1-phenylethanol feed vaporization facilities and product vapour condensing facilities. As 1-phenylethanol containing feedstock was used a sample of the process stream to the styrene reactor system of a commercial Propylene Oxide/Styrene Monomer plant. The feedstock contained 78.9 wt.% of 1-phenylethanol, 4.5 wt.% of 2-phenylethanol, 15.6 wt.% of methylphenylketone. The remainder up to 100% consisted of water and impurities and (by)products of the preceding oxidation and epoxidation sections. The outlet stream of the microflow unit was liquefied by condensation and the resulting two-phase liquid system was analyzed by means of gas chromatographic analysis.

The dehydration experiment was carried out at test conditions of 1.0 bara pressure and a temperature of 300 °C. The feed rate of the 1-phenylethanol containing feedstock was maintained at 30 grams per hour and the reactor tube was loaded with 20 cm³ of catalyst.

Activity (conversion) and reaction selectivity of the catalyst were determined from the gas chromatographic analyses of reaction product samples. In Table 2 the conversion rates for both 1-phenylethanol and 2-phenylethanol and the selectivity rates after the beginning of the reaction (t = 8 hours) and after the reaction has proceeded for some time and the catalyst has aged (t = 70 hours), are indicated.

**Table 1**

| **Catalyst** | **A** | **B** | **C(*)** | **D(*)** |
|---|---|---|---|---|
| type of pore size distribution | multimodal (Fig. 2) | multimodal (Fig. 3) | monomodal (Fig. 4) | monomodal (Fig. 5) |
| MPDᵥ (nm) | 19.7 | 27.6 | 15.0 | 18.2 |
| pore diameter (nm) at highest peak in range of 0-100 nm | 14.2 | 18.2 | 14.2 | 17.3 |
| pore diameter (nm) at highest peak in range of > 100 nm | 576.0 | 480.0 | no peaks | no peaks |
| total pore volume (ml/g) | 1.04 | 0.92 | 0.70 | 0.63 |
| % of total pore volume in pores having a diameter of 0-100 nm | 71.2 | 70.4 | 98.4 | 96.3 |
| % of total pore volume in pores having a diameter of > 100 nm | 28.8 | 29.6 | 1.6 | 3.7 , |
| surface area (m²/g) | 132.0 | 89.3 | 126.0 | 98.6 |
| particle shape | rod-like; hollow quadrilobal cross-section | solid cylinder | solid cylinder | solid cylinder |
| particle length (mm) | 5.4 | 5 | 5 | 5 |
| particle diameter (mm) | 5.9 / 5.0 (1) | 5 | 5 | 5 |
| particle bore diameter (mm) | 1.5 | no bore | no bore | no bore |
| side crushing strength (N) | 84 | 55 | 104 | 119 |
| bulk crushing strength (MPa) | 1.1 | 1.2 | > 1.6 | > 1.6 |

| | | | | |
|---|---|---|---|---|
| (*) = comparative catalyst; MPDᵥ = median pore diameter calculated by volume; (1) = both include the bore; the second does not include the quadrilobal extensions | | | | |

**Table 2**

| **Catalyst** | **A** | | **B** | | **C(*)** | | **D(*)** | |
|---|---|---|---|---|---|---|---|---|
| conversion of X (%) | X=1PE | X=2PE | X=1PE | X=2PE | X=1PE | X=2PE | X=1PE | X=2PE |
| at t = 8 h | 99.8 | 53.1 | 98.8 | 45.8 | 98.7 | 52.0 | 98.6 | 50.5 |
| at t = 70 h | 99.5 | 45.7 | 97.7 | 36.5 | 86.1 | 30.1 | 85.9 | 29.4 |
| % decrease | 0.3 | 13.9 | 1.1 | 20.3 | 12.8 | 42.1 | 12.9 | 41.8 |
| selectivity (%) | | | | | | | | |
| at t = 8 h | 94.9 | | 95.9 | | 95.3 | | 94.9 | |
| at t = 70 h | 95.4 | | 96.5 | | 94.9 | | 95.0 | |

| | | | | | | | | |
|---|---|---|---|---|---|---|---|---|
| 1PE = 1-phenylethanol; 2PE = 2-phenylethanol | | | | | | | | |

The above results from Table 2 demonstrate a considerable improvement in the activity of aged multimodal over aged monomodal alumina catalysts, in the conversion of both 1-phenylethanol and 2-phenylethanol. In addition, the selectivity was maintained at a high level.

## Claims

1. Process for the preparation of styrene and/or a substituted styrene from a feed containing 1-phenylethanol and 2-phenylethanol and/or a substituted 1-phenylethanol and a substituted 2-phenylethanol, comprising a gas phase dehydration of the feed at elevated temperature in the presence of a catalyst comprising particles of alumina having a multimodal pore size distribution.

2. Process according to claim 1, wherein the maximum in a first pore size range is at a pore diameter of from 5 to 30 nm and the maximum in a second pore size range is at a pore diameter of from 300 to 1,000 nm.

3. Process according to claim 1, wherein the pore diameters corresponding to the maximums in first and second pore size ranges are separated by at least 200 nm and by at most 1,000 nm.

4. Process according to any one of the preceding claims, wherein the median pore diameter calculated by volume (MPDᵥ) is from 5 to 50 nm.

5. Process according to any one of the preceding claims, wherein the MPDᵥ is greater than the pore diameter maximum in a first pore size range and smaller than the pore diameter maximum in a second pore size range.

6. Process according to any one of the preceding claims, wherein the total pore volume of the catalyst is of from 0.25 to 1.50 ml/g.

7. Process according to any one of the preceding claims, wherein the catalyst has from 10 to 40% of the total pore volume in pores having a diameter greater than 100 nm, and from 60 to 90% of the total pore volume in pores having a diameter from 2 to 100 nm.

8. Process according to any one of the preceding claims, wherein the catalyst has a surface area in the range of from 60 to 160 m²/g.

9. Process according to any one of the preceding claims, wherein the catalyst is a hollow quadrilobal shaped catalyst.

## Patentansprüche

1. Verfahren zur Herstellung von Styrol und/oder einem substituierten Styrol aus einer Beschickung, enthaltend 1-Phenylethanol und 2-Phenylethanol und/oder ein substituiertes 1-Phenylethanol und ein substituiertes 2-Phenylethanol, umfassend eine Gasphasendehydration der Beschickung bei erhöhter Temperatur in Gegenwart eines Katalysators, umfassend Aluminiumoxidpartikel mit multimodaler Porengrößenverteilung.

2. Verfahren nach Anspruch 1, wobei das Maximum eines ersten Porengrößenbereichs bei einem Porendurchmesser von 5 bis 30 nm liegt und das Maximum eines zweiten Porengrößenbereichs bei einem Porendurchmesser von 300 bis 1.000 nm liegt.

3. Verfahren nach Anspruch 1, wobei die Porendurchmesser, die den Maximua der ersten und zweiten Porengrößenbereiche entsprechen, um mindstens 200 nm und um höchstens 1.000 nm auseinander liegen.

4. Verfahren nach einem der vorhergehenden Ansprüche, wobei der mittlere, auf der Grundlage des Volumens berechnete Porendurchmesser (MPDᵥ) 5 bis 50 nm beträgt.

5. Verfahren nach einem der vorhergehenden Ansprüche, wobei der MPDᵥ größer als der maximale Porendurchmesser in einem ersten Porengrößerenbereich und kleiner als der maximale Porendurchmesser in einem zweiten Porengrößenbereich ist.

6. Verfahren nach einem der vorhergehenden Ansprüche, wobei das gesamte Porenvolumen des Katalysators 0,25 bis 1,50 ml/g beträgt.

7. Verfahren nach einem der vorhergehenden Ansprüche, wobei der Katalysator 10 bis 40 % des gesamten Porenvolumens bei Poren mit einem Durchmesser von über 100 nm aufweist und 60 bis 90 % des gesamten Porenvolumens bei Poren mit einem Durchmesser von 2 bis 100 nm aufweist.

8. Verfahren nach einem der vorhergehenden Ansprüche, wobei der Katalysator eine Oberfläche im Bereich von 60 bis 160 m²/g aufweist.

9. Verfahren nach einem der vorhergehenden Ansprüche, wobei der Katalysator ein hohler Katalysator mit quadrilobaler Form ist.

## Revendications

1. Procédé de préparation de styrène et/ou de styrène substitué à partir d'une charge contenant du 1-phényléthanol et du 2-phényléthanol et/ou un 1-phényléthanol substitué et un 2-phényléthanol substitué, comprenant une déshydratation en phase gazeuse de la charge à température élevée en présence d'un catalyseur comprenant des particules d'alumine ayant une distributionmultimodale de tailles de pores.

2. Procédé selon la revendication 1, dans lequel le maximum dans une première plage de tailles de pores se situe à un diamètre de pores de 5 à 30 nm et le maximum dans une seconde plage de tailles de pores se situe à un diamètre de pores de 300 à 1000 nm.

3. Procédé selon la revendication 1, dans lequel les diamètres de pores correspondant aux maxima dans les première et seconde plages de tailles de pores sont séparés d'au moins 200 nm et au plus de 1000 nm.

4. Procédé selon l'une quelconque des revendications précédentes, dans lequel le diamètre de pores médian calculé en volume (MPDᵥ) est de 5 à 50 nm.

5. Procédé selon l'une quelconque des revendications précédentes, dans lequel le MPDᵥ est supérieur au maximum de diamètre de pores dans une première plage de tailles de pores et inférieur au maximum de diamètre de pores dans une seconde plage de tailles de pores.

6. Procédé selon l'une quelconque des revendications précédentes, dans lequel le volume de pores total du catalyseur est de 0,25 à 1,50 ml/g.

7. Procédé selon l'une quelconque des revendications précédentes, dans lequel le catalyseur possède 10 à 40 % du volume de pores total en pores d'un diamètre supérieur à 100 nm et 60 à 90 % du volume de pores total en pores d'un diamètre de 2 à 100 nm.

8. Procédé selon l'une quelconque des revendications précédentes, dans lequel le catalyseur a une surface spécifique dans la plage de 60 à 160 m²/g.

9. Procédé selon l'une quelconque des revendications précédentes, dans lequel le catalyseur est un catalyseur creux de forme quadrilobée.
